(19) Europäisches Patentamt
European Patent Office
Office européen des brevets

(11) Publication number: **0 596 465 A1**

(12) **EUROPEAN PATENT APPLICATION**

(21) Application number: **93117800.8**

(51) Int. Cl.$^5$: **A61K 7/032**, C09C 1/26

(22) Date of filing: **03.11.93**

(30) Priority: **04.11.92 JP 317976/92**

(43) Date of publication of application:
**11.05.94 Bulletin 94/19**

(84) Designated Contracting States:
**DE FR IT**

(71) Applicant: **MITSUBISHI PENCIL KABUSHIKI KAISHA**
**23-37, Higashi Ohio 5-chome**
**Shinagawa-ku, Tokyo 140(JP)**

(72) Inventor: **Kirita, Kazuhisa**
**1106-4, Shimoshinden,**
**Tamamura-cho**
**Sawa-gun, Gunma(JP)**
Inventor: **Chikatsune, Keizo**
**1-12-14, Miyahara,**
**Yodogawa-ku**
**Osaka-shi, Osaka(JP)**

(74) Representative: **VOSSIUS & PARTNER**
**Siebertstrasse 4**
**D-81675 München (DE)**

(54) **Liner cosmetics.**

(57) Oily reservoir type eye liner cosmetics are here disclosed which comprise, as essential components, 35-95 wt% of a light liquid iso-paraffin having 8 to 15 carbon atoms, 3-40 wt% of a Berlin blue having a particle diameter of 0.2 µm or less, and 2-40 wt% of a dispersant which is a mixture of three components, each of which is selected from each of the three groups of (A) a betaine-based surface active agent or the like, (B) a polyoxyethylene-based nonionic surface active agent or the like, and (C) a lecithin or an N-acylamino acid.

According to the present invention, good preservation can be obtained, even if any particular preservative is not used, and therefore troubles such as irritation and allergy due to the blend of the preservative are not present. Water resistance is also good. In addition, since the Berlin blue having a particle diameter of 0.2 µm or less and the specific dispersants are used, dispersion stability can be improved, and hue change which tends to occur owing to the sedimentation of the pigment at a nib can be prevented.

EP 0 596 465 A1

The present invention relates to cosmetics, and more particularly, it relates to reservoir type eye liner cosmetics.

An eye liner having a reservoir type sign pen structure is handier and easier to write a thin line as compared with a bottle type eye liner, and this reservoir type sign pen structure is preferable as a container for the eye liner. The eye liners have heretofore been on the market, but in all of the eye liners, aqueous cosmetics have been used. The aqueous cosmetics used in the conventional reservoir type eye liners have viscosities in the range which the structure permits, and some of the aqueous cosmetics contain a pigment having a particle diameter of about 0.2 μm or less for the sake of the dispersion stabilization of the pigment. However, most of the aqueous cosmetics are poor in water resistance, adhesive properties and preservation. In a nib of sliver fibers which can be used in the reservoir type eye liner, the effect of a preservative can scarcely be originally exerted, and if it is attempted to solve this problem by the aqueous cosmetics, the amount of the preservative to be blended increases, so that the probability of irritation and allergy to skin tends to heighten. In order to improve water resistance and adhesive properties, an O/W emulsification technique and a resin emulsion addition technique have been utilized for the eye liners except the reservoir type, but the former technique cannot control viscosity to 50 mPa•s or less and the latter technique is not suitable for the reservoir type in which parts such as the reservoir and the nib having a large surface area are used.

An object of the present invention is to provide an oily reservoir type eye liner which has good pigment dispersion properties and which are more excellent in preservation and water resistance than conventional reservoir type eye liner cosmetics.

This object is solved by a light liquid iso-paraffin having a specific number of carbon atoms as a main solvent, a Berlin blue which can be obtained by a reaction of a ferrocyanide and a ferric salt or a ferricyanide and a ferrous salt and which has a particle diameter of 0.2 μm or less, and a mixture of up to three surface active agents as a dispersant.

Now, the present invention will be described in detail.

As a main solvent, a light liquid iso-paraffin having preferably 8 to 15 carbon atoms, more preferably 11 to 12 carbon atoms is used. The light liquid iso-paraffin is more excellent in drying characteristics than a liquid paraffin. The amount of the light liquid iso-paraffin to be blended is preferably in the range of 35 to 95 wt%.

As a pigment, a berlin blue having a particle diameter of 0.2 μm or less obtainable by a reaction of a ferrocyanide and a ferric salt or a ferricyanide and a ferrous salt is used. A berlin blue obtained by an oxidizing process is in general not suitable for the present invention, because of usually having a large particle diameter. Furthermore, if the particle diameter of the berlin blue is larger than 0.2 μm, the sedimentation and separation of the pigment occur in a reservoir and a nib, so that hue change takes place at the time of application. The amount of the pigment to be blended is preferably in the range of 3 to 40% by weight.

As a dispersant, there may be used a mixture of three components, each of which is selected from each of the following three groups A, B and C:

A. an aminoacetic acid betaine-based surface active agent, an aminocarboxylic acid-based surface active agent, and an imidazoline-based surface active agent,

B. a polyoxyethylene alkyl ether phosphoric acid, a polyoxyethylene alkyl ether, a polyoxyethylene alkylphenyl ether, a polyethylene glycol fatty acid ester, a sucrose fatty acid ester and an ethylene oxide adduct thereof, a glycerin fatty acid ester and an ethylene oxide adduct thereof, a sorbitan fatty acid ester and an ethylene oxide adduct thereof, and

C. a lecithin and an N-acylamino acid.

With regard to the group A, the aminoacetic acid betaine-based surface active agent can be represented by the formula

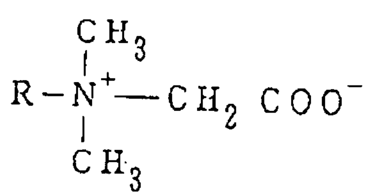

wherein the carbon number of R is preferably in the range of 12 to 24.

The aminocarboxylic acid-based surface active agent can be represented by the formula

$R-NH-(CH_2)_n-COOH$

wherein the carbon number of R is preferably in the range of 12 to 24, and n is preferably from 1 to 3.

The imidazoline-based surface active agent can be represented by the formula

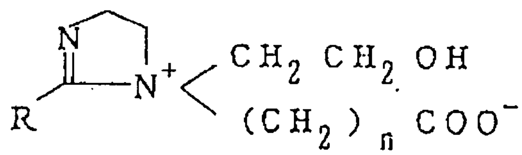

wherein the carbon number of R is preferably in the range of 12 to 24, and n is preferably from 1 to 3.

The dispersants in the group A can be used singly or in combination as the group A, and the amount of the dispersants in the group A is preferably from 10 to 40 wt% in the total dispersants.

The dispersants in the group B are used singly or in combination, and the HLB of these dispersants is preferably adjusted to the range of 3 to 8. The amount of the dispersants in the group B is preferably from 30 to 50 wt% in the total dispersants.

With regard to the group C, the N-acylamino acid surface active agent can be represented by the formula

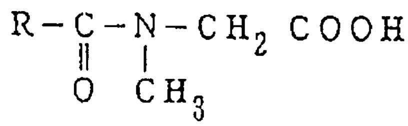

wherein the carbon number of R is preferably in the range of 12 to 18. The dispersants in the group C are used singly or in combination, and the amount of the dispersants in the group C is preferably from 10 to 40 wt% in the total dispersants.

The total amount of the dispersants is preferably in the range of 2 to 40% by weight in the cosmetics, and the weight ratio of the pigment to the dispersants is preferably in the range of 1:0.5 to 1:1.5.

In the cosmetics of the present invention, the above-mentioned requirements should be met and the viscosity should preferably be adjusted to 50 mPa•s or less.

In the present invention, other additives can be optionally used, so long as they do not impair dispersion stability, and examples of the additives include an antioxidant, a viscosity modifier, a protective agent for skin and a solvent.

The reservoir type eye liner cosmetics comprising the above-mentioned components can be prepared as follows.

A Berlin blue can be obtained by the reaction of a ferrocyanide and a ferric salt or a ferricyanide and a ferrous salt. Its typical example is a reaction of potassium ferrocyanide and ferric sulfate, potassium ferricyanide and ferrous sulfate, or sodium ferrocyanide and ferric sulfate and ammonium sulfate.

By the above-mentioned reaction, the Berlin blue is precipitated in water, and the addition of water and concentration are repeated by the use of decantation, centrifugal separation, pressure osmometry to wash the Berlin blue. Afterward, the dispersant of the group A is added thereto with stirring. After sufficient stirring, a means such as filtration or centrifugal separation is reaction of a ferrocyanide and a ferric salt or a ferricyanide and a ferrous salt is used and the dispersants described in Claim 2 are used, dispersion stability can be improved, and hue change which tends to occur owing to the sedimentation of the pigment at a nib can be prevented.

## EXAMPLES

Now, the present invention will be described in more detail in reference to examples.

Preparation Example 1

A 10 wt% aqueous solution of potassium ferrocyanide•trihydrate and a 6.5 wt% aqueous solution of ferric sulfate•n-hydrate (n = 6-9) were prepared, and 1 part by weight of the latter were gradually added to 2 parts by weight of the former with vigorously stirring form a Berlin blue. A small amount of the resultant reaction solution was sampled, and the aqueous potassium ferrocyanide solution and the aqueous ferric sulfate solution were added to a filtrate of the sampled solution. At this time, completion of the reaction could be confirmed from the fact that the filtrate was not colored. Water was added to the reaction solution, followed by centrifugal separation, to remove a supernatant liquid, and this operation was then repeated several times. Next, the solution was washed and then concentrated under heating to obtain an aqueous Berlin blue dispersion having a Berlin blue concentration of 12 wt%.

Preparation Example 2

| | | |
|---|---|---|
| 1. | Aqueous Berlin blue dispersion of Preparation Example 1 | 91.2 wt% |
| 2. | Lauryldimethylaminoacetic acid betaine (Nikko Chemicals Co., Ltd., AM-301, effective component 35%) | 8.8 wt% |
| | | 100.0 wt% |

The component 2 was added to the component 1, while the component 1 was stirred, and after the addition, the solution was further stirred for 60 minutes. Afterward, the solution was centrifugally separated and then heated to remove water therefrom, thereby obtaining dispersant-adsorbed powder. At this time, a pigment content in the dispersant-adsorbed powder was 80.5 wt%.

Preparation Example 3

| | | |
|---|---|---|
| 1. | Aqueous Berlin blue dispersion of Preparation Example 1 | 90.9 wt% |
| 2. | Laurylcarboxymethylhydroxyethylimidazolinium betaine (Kao Soap Co., Ltd., Anhitol 20Z, effective component 30%) | 9.1 wt% |
| | | 100.0 wt% |

The same procedure as in Preparation Example 2 was carried out to obtain dispersant-adsorbed powder. At this time, a pigment content in the dispersant-adsorbed powder was 83.0 wt%.

Preparation Example 4

| | | |
|---|---|---|
| 1. | Aqueous Berlin blue dispersion of Preparation Example 1 | 97.6 wt% |
| 2. | Laurylaminopropionic acid (Henkel Hakusui Co., Ltd., Delifat 151C, effective component 40%) | 3.4 wt% |
| | | 100.0 wt% |

The same procedure as in Preparation Example 2 was carried out to obtain dispersant-adsorbed powder. At this time, a pigment content in the dispersant-adsorbed powder was 84.2 wt%.

Example 1

| | | |
|---|---|---|
| 1. | Dispersant-adsorbed powder of Preparation Example 2 | 32.0 wt% |
| 2. | Sorbitan sesquioleate (HLB 3.7, Nikko Chemicals Co., Ltd., SO-15) | 7.0 wt% |
| 3. | P.O.E. (5) nonyl phenyl ether (HLB 8.0, Nikko Chemicals Co., Ltd., NP-5) | 2.0 wt% |
| 4. | Soybean phospholipid (The Nisshin Oil Mills, Ltd., Basis LP-20) | 4.0 wt% |
| 5. | Light liquid iso-paraffin (Nippon Shell Chemicals Co., Ltd., Shellsol 71) | 55.0 wt% |
| | | 100.0 wt% |

The above-mentioned materials 2 to 5 were mixed and dissolved, and the material 1 was added thereto. Next, the solution was stirred for 60 minutes, and then subjected to centrifugal separation to remove coarse particles therefrom, thereby obtaining cosmetics. The viscosity and the average particle diameter of the thus obtained cosmetics were 23 mPa•s (cp) and 0.12 $\mu$m, respectively.

Example 2

| | | |
|---|---|---|
| 1. | Dispersant-adsorbed powder of Preparation Example 3 | 25.0 wt% |
| 2. | P.O.E. (2) alkyl ether phosphoric acid (HLB 6.5, Nikko Chemicals Co., Ltd., DDP-2) | 2.0 wt% |
| 3. | P.O.E. (2) oleyl ether (HLB 7.5, Nikko Chemicals Co., Ltd., BO-2) | 4.0 wt% |
| 4. | P.O.E. (4) monostearate (HLB 6.5, Nikko Chemicals Co., Ltd., MYS-4) | 1.0 wt% |
| 5. | Oleoylsarcosine (Nikko Chemicals Co., Ltd., Sarcosinate OH) | 5.0 wt% |
| 6. | Light liquid iso-paraffin (Nippon Shell Chemicals Co., Ltd., Shellsol 71) | 61.0 wt% |
| | | 100.0 wt% |

The above-mentioned materials 2 to 6 were mixed and dissolved, and the material 1 was added thereto. Next, the solution was stirred for 60 minutes, and then subjected to centrifugal separation to remove coarse particles therefrom, thereby obtaining cosmetics. The viscosity and the average particle diameter of the thus obtained cosmetics were 16 mPa•s and 0.10 $\mu$m, respectively.

Example 3

| | | |
|---|---|---|
| 1. | Dispersant-adsorbed powder of Preparation Example 4 | 36.0 wt% |
| 2. | Glycerin monostearate (HLB 4.0, Nikko Chemicals Co., Ltd., MGS-A) | 4.0 wt% |
| 3. | Sorbitan monooleate (HLB 4.3, Nikko Chemicals Co., Ltd., SO-10) | 8.0 wt% |
| 4. | Soybean phospholipid (The Nisshin Oil Mills, Ltd., Basis LP-20) | 7.0 wt% |
| 5. | Light liquid iso-paraffin (Nippon Shell Chemicals Co., Ltd., Shellsol 71) | 45.0 wt% |
| | | 100.0 wt% |

The above-mentioned materials 2 to 5 were mixed and dissolved, and the material 1 was added thereto. The solution was stirred for 60 minutes, and then subjected to centrifugal separation to remove coarse particles therefrom, thereby obtaining cosmetics. The viscosity and the average particle diameter of the thus obtained cosmetics were 21 mPa•s and 0.10 $\mu$m, respectively.

Comparative Preparation Example 1

| | | |
|---|---|---|
| 1. | Berlin blue (DainichiSeika Color & Chemicals MFG. Co., Ltd., No. 650) | 12.0 wt% |
| 2. | Purified water | 88.0 wt% |
| | | 100.0 wt% |

The above-mentioned material 1 was added to the material 2, while the material 2 was stirred, followed by stirring for 60 minutes, to obtain an aqueous dispersion of the commercially available Berlin blue.

Comparative Example 1

The same procedure as in Preparation Example 2 and Example 1 was carried out except that a Berlin blue of Preparation Example 1 was replaced with the aqueous dispersion of a commercially available Berlin blue of Comparative Example 1, thereby obtaining cosmetics. The viscosity and the average particle diameter of the thus obtained cosmetics were 14 mPa•s and 0.24 $\mu$m, respectively. It should be noted that the particle diameter of the cosmetics was more than 0.2 $\mu$m.

Comparative Example 2

The same procedure as in Preparation Example 3 and Example 2 was carried out except that a Berlin blue of Preparation Example 1 was replaced with the aqueous dispersion of a commercially available Berlin blue of Comparative Preparation Example 1, thereby obtaining cosmetics. The viscosity and the average particle diameter of the thus obtained cosmetics were 15 mPa•s and 0.30 $\mu$m, respectively. It should be noted that the particle diameter of the cosmetics was more than 0.2 $\mu$m.

Comparative Preparation Example 2

| | | |
|---|---|---|
| 1. | Aqueous Berlin blue dispersion of Comparative Example 1 | 97.3 wt% |
| 2. | Lauryldimethylaminoacetic acid betaine (Nikko Chemicals Co., Ltd., AM-301, effective component 35%) | 2.7 wt% |
| | | 100.0 wt% |

The same procedure as in Preparation Example 2 was carried out to obtain dispersant-adsorbed powder. At this time, a pigment content in the dispersant-adsorbed powder was 94.5 wt%. The ratio of the dispersant to the pigment was 0.23:1. That is, the ratio of the dispersant to the pigment was less than 0.5.

Comparative Example 3 (example in which the balance of dispersants A, B and C was bad)

| | | |
|---|---|---|
| 1. | Dispersant-adsorbed powder of Comparative Preparation Example 2 | 31.0 wt% |
| 2. | Sorbitan sesquioleate | 3.5 wt% |
| 3. | P.O.E. (5) nonyl phenyl ether | 3.3 wt% |
| 4. | Soybean phospholipid | 5.5 wt% |
| 5. | Light liquid iso-paraffin | 56.7 wt% |
| | | 100.0 wt% |

It was tried to obtain cosmetics by the same procedure as in Example 1, but the pigment cohered, so that good dispersion could not be obtained. The ratios of the dispersant A, B and C to the total dispersants were less than 10 wt%, more than 50 wt%, and more than 40 wt%, respectively.

Comparative Preparation Example 3

| | | |
|---|---|---|
| 1. | Aqueous Berlin blue dispersion of Comparative Example 1 | 97.7 wt% |
| 2. | Laurylcarboxymethylhydroxyethylimidazolinium betaine (Kao Soap Co., Ltd., Anhitol 20Z, effective component 30%) | 2.3 wt% |
| | | 100.0 wt% |

The same procedure as in Preparation Example 2 was carried out to obtain dispersant-adsorbed powder. At this time, a pigment content in the dispersant-adsorbed powder was 95.4% by weight.

Comparative Example 4 (example in which dispersants were enough as a whole but the balance of a dispersant A was bad)

| | | |
|---|---|---|
| 1. | Dispersant-adsorbed powder of Comparative Preparation Example 3 | 21.2 wt% |
| 2. | P.O.E. (2) alkyl ether phosphoric acid | 3.0 wt% |
| 3. | P.O.E. (2) oleyl ether | 5.0 wt% |
| 4. | P.O.E. (4) monostearate | 1.9 wt% |
| 5. | Oleoylsarcosine | 3.4 wt% |
| 6. | Light liquid iso-paraffin | 65.5 wt% |
| | | 100.0 wt% |

It was tried to obtain cosmetics by the same procedure as in Example 2, but the ratio of the dispersant A to the dispersants B + C was less than 1/9, though a ratio of at least 1/9 was necessary. Thus, the pigment cohered, so that good dispersion could not be obtained.

Comparative Example 5 (example in which dispersants were enough as a whole but the balance of a dispersant B was bad)

| | | |
|---|---|---|
| 1. | Dispersant-adsorbed powder of Comparative Preparation Example 2 | 32.0 wt% |
| 2. | Sorbitan sesquioleate | 3.0 wt% |
| 3. | P.O.E. (5) nonyl phenyl ether | 1.8 wt% |
| 4. | Soybean phospholipid | 6.2 wt% |
| 5. | Light liquid iso-paraffin | 57.0 wt% |
| | | 100.0 wt% |

The same procedure as in Example 1 was carried out to obtain cosmetics. The viscosity and the average particle diameter of the thus obtained cosmetics were 30 cp and 0.26 µm, respectively, and it should be noted that this diameter was more than 0.2 µm. The content of the dispersant B was 4.8 wt%, which was less than 30 wt%.

Comparative Example 6 (example in which dispersants were enough as a whole but the balance of a dispersant C was bad)

| | | |
|---|---|---|
| 1. | Dispersant-adsorbed powder of Preparation Example 2 | 32.0 wt% |
| 2. | Sorbitan sesquioleate | 9.0 wt% |
| 3. | P.O.E. (5) nonyl phenyl ether | 3.5 wt% |
| 4. | Soybean phospholipid | 1.5 wt% |
| 5. | Light liquid iso-paraffin | 54.0 wt% |
| | | 100.0 wt% |

The same procedure as in Example 1 was carried out to obtain cosmetics. The viscosity and the average particle diameter of the thus obtained cosmetics were 29 mPa•s and 0.29 µm, respectively, and hence it should be noted that this diameter was more than 0.2 µm. The content of the dispersant C in the total dispersants was less than 10 wt% (the dispersant B content was also more than 50 wt%).

Comparative Example 7 (example in which the HLB of a dispersant B was low)

| | | |
|---|---|---|
| 1. | Dispersant-adsorbed powder of Preparation Example 2 | 32.0 wt% |
| 2. | Sorbitan tristearate (HLB 2.1, Nikko Chemicals Co., Ltd., SS-30) | 8.0 wt% |
| 3. | P.O.E. (1) monostearate (HLB 2.0, Nikko Chemicals Co., Ltd., MYS-1) | 4.0 wt% |
| 4. | Soybean phospholipid | 6.5 wt% |
| 5. | Light liquid iso-paraffin | 49.5 wt% |
| | | 100.0 wt% |

It was tried to obtain cosmetics by the same procedure as in Example 1, but the HLB of the dispersant B was 2.05, so that the pigment cohered and good dispersion could not be obtained.

Comparative Example 8 (example in which the HLB of a dispersant B was high)

| | | |
|---|---|---|
| 1. | Dispersant-adsorbed powder of Preparation Example 2 | 32.0 wt% |
| 2. | P.O.E. (6) sorbitan monolaurate (HLB 13.3, Kao Soap Co., Ltd., Leodol TW-106) | 8.0 wt% |
| 3. | P.O.E. (10) cetyl ether (HLB 13.5, Nikko Chemicals Co., Ltd., BC-10) | 4.0 wt% |
| 4. | Soybean phospholipid | 6.5 wt% |
| 5. | Light liquid iso-paraffin | 49.5 wt% |
| | | 100.0 wt% |

It was tried to obtain cosmetics by the same procedure as in Example 1, but the HLB of the dispersant B was 13.4, so that the pigment cohered and good dispersion could not be obtained.

Comparative Example 9 (example of aqueous cosmetics)

An aqueous Berlin blue dispersion of Example 1 was further concentrated to obtain an aqueous Berlin blue dispersion having a Berlin blue concentration of 25 wt%.

| | | |
|---|---|---|
| 1. | 25 wt% aqueous Berlin blue dispersion | 75.00 wt% |
| 2. | Sodium dioctylsulfosuccinate (Nikko Chemicals Co., Ltd., OTP100) | 10.00 wt% |
| 3. | P.O.E. (15) oleyl ether (Nikko Chemicals Co., Ltd., BO-15) | 8.00 wt% |
| 4. | 1,3-butylene glycol | 6.65 wt% |
| 5. | Methyl paraoxybenzoate | 0.25 wt% |
| 6. | Propyl paraoxybenzoate | 0.10 wt% |
| | | 100.0 wt% |

The above-mentioned material 2 was added to the material 1, while the material 1 was stirred, and the mixture was then stirred for 60 minutes. Afterward, the material 3 was added thereto, followed by stirring for 60 minutes. A solution obtained by previously mixing the materials 4, 5 and 6 was added to the above-mentioned solution, and stirring was then carried out for 10 minutes. Next, coarse particles were removed from the solution by centrifugal separation to obtain aqueous cosmetics. The viscosity and the average particle diameter of the thus obtained cosmetics were 16 mPa•s and 0.10 µm, respectively.

With regard to the above-mentioned examples and comparative examples, evaluation and estimation were done by the following evaluation procedure.

1. Test of preservation effect in accordance with U.S.P. XXI (in Table 1, the preservation effect is represented by the number of bacteria of all kinds/g after 3 days).
2. Measurement of bacteria number at a nib after a reservoir type eye liner container was used for 2 weeks.
3. Water resistance: A sample was applied onto the back of a hand, and it was then evaluated whether or not the water resistance was present.
4. Dispersion stability: Reservoir type eye liner containers were packed with samples, and some of the containers were allowed to stand upward and the remaining containers were done downward at a

temperature of 25°C for 1 month. Afterward, the densities of lines written by these eye liners were compared, and the evaluation was made by the utilization of the fact that the better a dispersion stability was, the smaller a density difference between the lines written by the upward and downward samples was.

The results are shown in Table 1.

Table 1

| | Evaluation | | | | Note |
|---|---|---|---|---|---|
| | 1 | 2 | 3 | 4 | |
| Example 1 | 10 bacteria/g or less | 10 bacteria/g or less | Good | Difference was not observed. | |
| Example 2 | 10 bacteria/g or less | 10 bacteria/g or less | Good | Difference was not observed. | |
| Example 3 | 10 bacteria/g or less | 10 bacteria/g or less | Good | Difference was not observed. | |
| Comp. Ex. 1 | 10 bacteria/g or less | 10 bacteria/g or less | Good | Difference was observed. | |
| Comp. Ex. 2 | 10 bacteria/g or less | 10 bacteria/g or less | Good | Difference was observed. | |
| Comp. Ex. 3 | - | - | - | - | Dispersion was bad. |
| Comp. Ex. 4 | - | - | - | - | Dispersion was bad. |

Table 1 (Continued)

| | Evaluation 1 | Evaluation 2 | Evaluation 3 | Evaluation 4 | Note |
|---|---|---|---|---|---|
| Comp. Ex. 5 | 10 bacteria/g or less | 10 bacteria/g or less | Good | Difference was observed. | |
| Comp. Ex. 6 | 10 bacteria/g or less | 10 bacteria/g or less | Good | Difference was observed. | |
| Comp. Ex. 7 | - | - | - | - | Dispersion was bad. |
| Comp. Ex. 8 | - | - | - | - | Dispersion was bad. |
| Comp. Ex. 9 | 10 bacteria/g or less | $4\times10^3$ bacteria/g | Good | Difference was not observed. | |

## Claims

1. Liner cosmetics comprising a light liquid iso-paraffin, a Berlin blue having a particle diameter of 0.2 $\mu$m or less and a dispersant as essential components.

**2.** The liner cosmetics according to claim 1, wherein said iso-paraffin is present in an amount of 20 - 95 wt%, the Berlin blue in an amount of 3 - 40 wt% and the dispersant in an amount of 2 - 40 wt%.

**3.** The liner cosmetics according to claim 1, wherein said iso-paraffin is present in an amount of 35 - 95 wt% and has 8 to 15 carbon atoms, the Berlin blue in an amount of 3 - 40 wt% and the dispersant in an amount of 2 - 40 wt% with the proviso that the amount of the above three components does not exceed 100 wt%.

**4.** The liner cosmetics according to any of claims 1 to 3, wherein said dispersant is a mixture of three components, each of which is selected from each of the following three groups A, B and C:

A. an aminoacetic acid betaine-based surface active agent, an aminocarboxylic acid-based surface active agent, and an imidazoline-based surface active agent,

B. a polyoxyethylene alkyl ether phosphoric acid, a polyoxyethylene alkyl ether, a polyoxyethylene alkylphenyl ether, a polyethylene glycol fatty acid ester, a sucrose fatty acid ester and an ethylene oxide adduct thereof, a glycerin fatty acid ester and an ethylene oxide adduct thereof, and a sorbitan fatty acid ester and an ethylene oxide adduct thereof, and

C. a lecithin and an N-acylamino acid.

**5.** The liner cosmetics according to Claim4, wherein said aminoacetic acid betaine-based surface active agent is represented by the formula

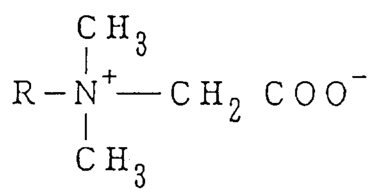

wherein the carbon number of R is in the range of 12 to 24.

**6.** The liner cosmetics according to Claim 4, wherein said aminocarboxylic acid-based surface active agent is represented by the formula

$R-NH-(CH_2)_n-COOH$

wherein the carbon number of R is in the range of 12 to 24, and n is from 1 to 3.

**7.** The liner cosmetics according to Claim 4, wherein said imidazoline-based surface active agent is represented by the formula

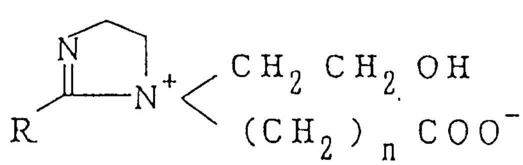

wherein the carbon number of R is in the range of 12 to 24, and n is from 1 to 3.

**8.** The liner cosmetics according to any of Claims 4 to 7, wherein the HLB of one or a mixture of two or more selected from said surface active agents in the group B is from 3 to 8.

**9.** The liner cosmetics according to Claim 4, wherein said N-acylamino acid-based surface active agent is represented by the formula

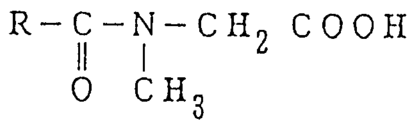

wherein the carbon number of R is in the range of 12 to 18.

**10.** The liner cosmetics according to any of Claims 4 to 9, wherein a blending ratio of the groups A, B and C in the total dispersants is
the group A: 10-40 wt%,
the group B: 30-50 wt%, and
the group C: 10-40 wt%;
provided that the sum of A, B and C is 100 wt%.

**11.** The liner cosmetics according to any of Claims 1 to 10, wherein viscosity is 50 mPa•s or less.

**12.** The liner cosmetics according to any of Claims 1 to 11, wherein a weight ratio of said pigment to said dispersant is in the range of 1:0.5 to 1:1.5.

European Patent Office

# EUROPEAN SEARCH REPORT

Application Number
EP 93 11 7800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-91 12793 (L'OREAL)<br>* the whole document * | 1-3 | A61K7/032<br>C09C1/26 |
| Y | FR-A-2 669 223 (MITSUBISHI PENCIL CO. LTD)<br>* the whole document * | 1-3 | |
| A | GB-A-2 216 797 (L'OREAL)<br>* page 5, line 10 - line 17; example 1 * | 1-3 | |
| P,A | FR-A-2 683 145 (L'OREAL)<br>* the whole document * | 1-9 | |

TECHNICAL FIELDS SEARCHED (Int.Cl.5)

A61K
C09C

The present search report has been drawn up for all claims

| Place of search | Date of completion of the search | Examiner |
|---|---|---|
| THE HAGUE | 7 February 1994 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)